Europäisches Patentamt

(19) European Patent Office

Office européen des brevets

(11) Publication number: **0 023 978**
**A2**

(12) # EUROPEAN PATENT APPLICATION

(21) Application number: **80104047.8**

(22) Date of filing: **12.07.80**

(51) Int. Cl.³: **A 61 K 7/00**

(30) Priority: **09.08.79 US 65255**

(43) Date of publication of application:
**18.02.81 Bulletin 81/7**

(84) Designated Contracting States:
**AT BE CH DE FR GB IT LI LU NL SE**

(71) Applicant: **BASF WYANDOTTE CORPORATION**
**1609 Biddle Avenue**
**Wyandotte Michigan 48192(US)**

(72) Inventor: **Schmolka, Irving Rudolf**
**21490 Parke Lane**
**Grosse Ile Michigan 48138(US)**

(74) Representative: **von Günner, Kurt, Dr. et al,**
**BASF Aktiengesellschaft Carl-Bosch-Strasse 38**
**D-6700 Ludwigshafen(DE)**

(54) Harmless cosmetic and toiletry products and method of making same.

(57) To avoid a problem of the development of carcinogenic nitrosamines in cosmetic and toiletry products which either contain triethanolamine or contain compounds which result when triethanolamine is used to neutralize an acidic substance, formulations are made in which there is used in place of the triethanolamine a suitable totally alkoxylated alkylene diamine, such as N,N,N',N'-tetrakis(2-hydroxypropyl)ethylene-diamine.

EP 0 023 978 A2

Croydon Printing Company Ltd

HARMLESS COSMETIC AND TOILETRY PRODUCTS TITLE MODIFIED
see front page

This invention relates to cosmetic and toiletry products, and in particular to ones which are similar to those which contain as ingredients an alkanolamine such as triethanolamine or a compound which results when such alkanolamine is used to neutralize an acidic substance. It has recently been discovered that the use of these alkanolamines in cosmetic and toiletry products is undesirable because of the triethanolamine's capability of forming a carcinogenic nitrosamine.

Many kinds of cosmetic and toiletry products are known, such as cuticle remover, hair dressing, sun screen lotion, brushless shave cream, moisturizing cream, baby cream, shampoo, face and hand cleaner lotion, barrier cream, and the like. Various recipes are known for products of each of the various kinds indicated above, in accordance with which compositions of matter suitable for each of the indicated purposes may be made. These compositions customarily contain ingredients such as water, natural or synthetic oils, fatty acids, fatty alcohols, fatty amides, fatty esters, anionic surfactants, non-ionic surfactants, polyhydric alcohols, perfumes, preservatives, inorganic salts, etc., the identity and proportions of the particular ingredients used in a particular case being determined by the properties needed in the product being made. It is common, in the formulation of such products, to use as an ingredient either triethanolamine or a compound which results when triethanolamine reacts with an acidic substance, i.e., a compound resulting from the use of triethanolamine as neutralizing agent. Moreover, before the making of the present invention, it had been discovered that triethanolamine (especially commercial grades of triethanolamine, which contain various amounts of diethanolamine) has the capability of forming nitrosamine compounds, and it had been discovered that such nitrosamine compounds are detectably carcinogenic. It may be taken as known at the time of the making of this invention that it would be desirable to find a replacement for triethanolamine which would not yield detectable amounts of nitrosamine compounds.

The compound N,N,N',N'-tetrakis(2-hydroxypropyl)-ethylenediamine and other similar totally hydroxy-propylated or hydroxy-alkylated alkylene diamines are known from U.S. Patent No. 2,697,118. That patent contains no information on the subject of the propensity of such compounds to form nitrosamines.

The prior art is similarly silent on the issue of whether, if N,N,N',N'-tetrakis(2-hydroxypropyl)ethylenediamine is substituted for an alkanolamine such as diethanolamine or triethanolamine, products having satisfactory performance characteristics for use as cosmetic and toiletry products can still be made.

the weight of triethanolamine previously used in formulating a similar product. In many instances, very satisfactory results may be obtained with a substitution on a weight-for-weight basis. It should be pointed out, however, that in instances in which the triethanolamine is used in a cosmetic or toiletry preparation particularly for its neutralizing effect, so that it is present in the final formulation in the form of a compound resulting from the neutralization of an acidic substance with triethanolamine, mere substitution on a weight-for-weight basis is sometimes likely not to be appropriate. In such cases, the use of the stoichiometrically equivalent amount of oxyalkylated alkylene diamine is indicated. This implies that one would, in calculating a stoichiometrically equivalent amount, take into account not only the respective molecular weights of triethanolamine and the oxyalkylated alkylene diamine being used, but also the consideration that triethanolamine contains one nitrogen atom and the alkylene diamine contains two nitrogen atoms.

Moreover, other considerations come into play. It may not always be necessary to achieve in the products according to this invention a final pH quite so high as that of the corresponding product which contains an ethanolamine or its neutralization product. The human skin is on the slightly acid side, and in many cases a product marginally more acidic than the corresponding known product is perfectly acceptable. For that matter, so is a product which is slightly less acidic

To avoid a problem of the development of carcinogenic nitrosamines in cosmetic and toiletry products which either contain triethanolamine or contain compounds which result when triethanolamine is used to neutralize an acidic substance, formulations are made in which there is used in place of the triethanolamine a suitable totally alkoxylated alkylene diamine, such as N,N,N',N'-tetrakis(2-hydroxypropyl)-ethylenediamine. The invention extends, moreover, to the similar replacement of diethanolamine or monoethanolamine.

In accordance with the invention, cosmetic and toiletry products are made which contain, in place of triethanolamine, a suitable totally hydroxyalkylated alkylene diamine. The suitable totally hydroxyalkylated alkylene diamines are ones that are preferably based on ethylene diamine or another alpha-omega alkylene diamine containing 2 to 6 carbon atoms. The alkylene oxide or oxides used in the making of the suitable totally hydroxyalkylated alkylene diamines used in accordance with this invention are ones containing at least 2 carbon atoms, preferably ones containing at least 3 carbon atoms and possibly 4 or more carbon atoms. Most commonly, propylene oxide is used, but the propylene oxide may be replaced in whole or in part by butylene oxide or other higher alkylene oxides, or by ethylene oxide.

In general, the suitably totally hydroxyalkylated alkylene diamine is used in place of the triethanolamine in an amount which is approximately 80 to 200 percent by weight of

**0023978**

or more basic, in many instances. There is also the consideration that the suitable totally alkoxylated diamines proposed for use in accordance with this invention are not so strongly basic as, for example, triethanolamine or diethanolamine. This implies that in some circumstances, one would use slightly more of them than the amount that would be predicted as being necessary in accordance with the usual stoichiometric calculations.

Cases will differ, of course, depending upon the identity and nature and quantity of the acidic substance to be neutralized; the amount arrived at by stoichiometric calculation may be perfectly appropriate in a situation in which a weakly acidic substance is to be neutralized, but the amount arrived at by stoichiometric calculations is less likely to be appropriate if the acid is a strong one.

Among those acids which can be neutralized, in whole or in part, with suitable oxyalkylated diamines in accordance with this invention are oleic, linoleic, lauric, myristic, behenic, stearic, palmitic, isostearic, and other similar fatty acids which are normally used in cosmetic and toiletry products. Also included are dicarboxylic acids such as the $C_{21}$ acid known as Westvaco diacid, as well as azelaic and sebacic acids. Citric, lactic, and other hydroxy organic acids, such as 12 hydroxy stearic acid are included, as well as the sulfuric or phosphoric acid esters of lauryl, myristyl, or similar fatty alcohols, and also sulfated or phosphated ethylene oxide adducts of lauryl and other fatty alcohols. Further included are organic sulfonic or sulfinic acids such as alkylbenzene or naphthalene sulfinic acid, in which the alkyl groups may vary from one carbon atom (methyl) to 15 carbon atoms (pentadecyl) and can be branched or straight-chain, as well as alkyl-substituted diphenylether sulfonic acids, such as DOWFAX, and iso-alpha-olefin sulfonic acids, in which the alpha-olefin varies from hexene-1 to octadecene-1 in chain length. Those skilled in the art will understand that the foregoing enumeration is to be considered as illustrative, and not in a limiting sense; other acids which suggest themselves as suitable for partial or total neutralization, in view of the foregoing enumeration or otherwise, may likewise be subjected to neutralization with N,N,N',N'-tetrakis(2-hydroxypropyl)ethylene diamine or the like in place of being neutralized with triethanolamine or diethanolamine, in the making of cosmetic or toiletry products in accordance with the present invention.

In any event, it is found that it is ordinarily possible to arrive at a product having satisfactory performance characteristics after a minimum of routine experimentation, starting with a weight-for-weight substitution and a substitution based upon a stoichiometrically equivalent amount. In that way, it is possible to arrive at an amount effective to yield the desired performance characteristics in the product made in accordance with the present invention.

The invention resides in the discovery that such a substitution, which may have seemed before the present to be of dubious usefulness and operability, both in respect to

0023978

O.Z. 2970/01049

obtaining products which still perform satisfactorily and in respect to obtaining products which do not produce nitrosamines detectable by 1979 methods, is generally both feasible and effective.

Except for the matters discussed above, the process of making and using the cosmetic or toiletry preparations that are made in accordance with the present invention is substantially the same as that of the corresponding preparation, made in accordance with the prior art.

In one aspect, the invention concerns making non-nitrosamine-forming cosmetic or toiletry preparations. This means that the compositions of such preparations must be free of other known nitrosamine precursors. In other words, it will do no good, if the objective is to obtain a cosmetic or toiletry preparation which does not form nitrosamines, to eliminate diethanol amine or triethanolamine as a nitrosamine precursor, if the composition of the cosmetic or toiletry preparation is one which contains other substances which form nitrosamines. In another, somewhat broader aspect of the invention, the objective is to obtain cosmetic or toiletry preparations which have satisfactory performance characteristics and a decreased tendency to form nitrosamines. In this regard, the knowledge provided by the present invention, to the effect that a suitable totally alkoxylated alkylene diamine may be substituted for triethanolamine or diethanolamine, is, in itself, a useful and unobvious concept, to the extent that the teachings of the present invention make it possible to overcome that part of the nitrosamine-formation problem which is attributable to the present usage of triethanolamine and/or diethanolamine, even in cosmetic or toiletry preparations which contain other ingredients which have been established as being nitrosamine precursors or will be established as being nitrosamine precursors in future research. It is impossible to predict at present what the specifications for cosmetic or toiletry products will be, in reference to the avoidance of nitrosamine formation, on the basis of public acceptance or pertinent legislation, but it is conceivable that the present invention, in its broad aspect of providing a cosmetic or toiletry preparation of reduced nitrosamine-forming tendency, will find use in instances in which the detectable nitrosamines in the product as formulated with the use of diethanolamine or triethanolamine are mainly attributable to the diethanolamine or triethanolamine, and when the invention in this broader aspect is practiced, there will be obtained a product which is found satisfactory, even though it contains some detectable amount of nitrosamines.

Thus, in accordance with a broad aspect of the present invention, there are obtained products of reduced nitrosamine-formation tendency and satisfactory performance characteristics, by the replacement of, for example, triethanolamine with N,N,N',N'-tetrakis(2-hydroxypropyl)ethylene diamine. In accordance with a narrower aspect of the present invention, there are obtained products which do not contain detectable amounts of nitrosamine and have satisfactory

0023978

O.Z. 2970/01049

performance characteristics, and such products are characterized by the absence of identified nitrosamine precursors.

An understanding of the invention may be aided by consideration of the following specific examples, in which parts are by weight unless otherwise specified. Substances referred to by commercial name in the Examples will be defined more particularly at the conclusion of the examples.

0023978

## Example 1

### Cuticle Remover

| | |
|---|---|
| VEEGUM | 1.00 |
| Water | 74.75 |
| Captan | 0.25 |
| QUADROL | 11.0 |
| Glycerine | 10.0 |
| LAURETH -4 | 1.2 |
| LAURETH -23 | 1.8 |
| | 100.0 |

## Example 2

### Sun Screen Lotion

| | |
|---|---|
| VEEGUM | 1.5 |
| Water | 77.5 |
| QUADROL | 5.0 |
| MYVACET 9-40 | 4.0 |
| Cetyl Alcohol | 0.5 |
| Stearic Acid | 1.5 |
| Cocoyl Sarcosine | 5.0 |
| p-aminobenzoic acid | 5.0 |
| Preservative | q.s. |
| | 100.0 |

0023978
O.Z. 2970/01049

## Example 3

### Hair Dressing

| | |
|---|---|
| CARBOPOL 934 | 0.5 |
| Water | 19.8 |
| QUADROL | 0.7 |
| Water | 35.0 |
| AMERCHOL L 101 | 5.0 |
| Isopropyl Lanolate | 1.0 |
| PEG 40 stearate | 3.0 |
| PPG 33 butyl ether | 15.0 |
| PPG 12 BUTETH 16 | 5.0 |
| Mineral Oil | 15.0 |
| Preservative | q.s. |
| Perfume | q.s. |
| | 100.0 |

## Example 4

### Brushless Shave Cream

| | |
|---|---|
| Stearic acid | 18.0 |
| Mineral oil | 5.0 |
| Polysorbate 60 | 5.0 |
| Sorbitol | 5.0 |
| Borax | 2.0 |
| QUADROL | 1.5 |
| Water | 63.5 |
| Preservative | q.s. |
| Perfume | q.s. |
| | 100.0 |

## Example 5

### Moisturizing Cream

| | |
|---|---|
| VEEGUM | 1.0 |
| Water | 72.5 |
| Glycerine | 4.0 |
| QUADROL | 1.5 |
| POLYPEPTIDE SF | 5.0 |
| Lanolin oil | 10.0 |
| Stearic acid | 2.0 |
| Isopropyl myristate | 2.0 |
| Cetyl alcohol | 2.0 |
| Perfume | q.s. |
| Preservative | q.s. |
| | 100.0 |

## Example 6

### Baby Cream

| | |
|---|---|
| Olive oil | 10.0 |
| Stearic acid | 4.0 |
| Lanolin | 3.0 |
| Glyceryl stearate | 12.0 |
| Sorbitol | 5.0 |
| QUADROL | 1.5 |
| Water | 64.5 |
| Preservative | q.s. |
| | 100.0 |

## Example 7

### Shampoo

| | |
|---|---|
| QUADROL | 3.5 |
| Alpha olefin sulfonate sodium salt (35%) | 52.0 |
| Oleic acid | 7.0 |
| Lauric diethanolamide | 2.0 |
| Water | 35.5 |
| | 100.0 |

## Example 8

### Face and Hand Cleaner Lotion

| | |
|---|---|
| VEEGUM | 2.5 |
| Water | 65.5 |
| QUADROL | 3.5 |
| Propylene glycol | 5.0 |
| Captan | 0.2 |
| Citric acid | 0.3 |
| Stearic acid | 4.0 |
| Cocoyl sarcosine | 6.0 |
| Cetyl alcohol | 1.0 |
| Glyceryl monostearate | 3.0 |
| Mineral oil | 5.0 |
| Lanolin oil | 4.0 |
| | 100.0 |

## Example 9

### Barrier Cream

| | |
|---|---|
| Mineral Spirits | 15.0 |
| Mineral oil | 15.0 |
| Water | 42.5 |
| PLURONIC 25R5 | 6.0 |
| Stearic acid | 7.5 |
| Silicone Fluid (350 centistokes) | 2.0 |
| Cetyl alcohol | 2.2 |
| QUADROL | 4.8 |
| Propylene Glycol | 5.0 |
| Preservative | q.s. |
| | 100.0 |

## Example 10

### Cream Lotion Shampoo

| | |
|---|---|
| Glyceryl monostearate | 3.0 |
| CARBOPOL 934 | 1.0 |
| QUADROL | 1.5 |
| QUADROL-neutralized dodecyl benzene sulfonate (60%) | 33.0 |
| Water | 61.5 |
| Perfume | q.s. |
| Preservative | q.s. |
| | 100.0 |

**0023978**

## Example 11

### Shampoo

| | |
|---|---:|
| QUADROL-neutralized lauraminopropionic acid | 6.0 |
| Lauraminopropionic acid | 3.0 |
| QUADROL-neutralized lauryl sulfate | 6.0 |
| Ethoxylated lanolin | 6.0 |
| Lauric isopropanolamide | 4.0 |
| Water | 75.0 |
| | 100.0 |

## Example 12

### Shampoo

| | |
|---|---:|
| QUADROL salt of cocoyl sarcosine | 10.0 |
| QUADROL-neutralized lauryl ether sulfate | 4.0 |
| Lauric isopropanolamide | 3.0 |
| Lauric diethanolamide | 3.0 |
| Ethylenediamine tetraacetic acid | 0.65 |
| Ethyl alcohol | 7.0 |
| Methyl cellulose | 0.75 |
| Water | 71.6 |
| Preservative | q.s. |
| Perfume | q.s. |
| | 100.0 |

## Example 13

### Shampoo

| | |
|---|---:|
| QUADROL-neutralized lauryl ether (3EO) sulfate (60% active) | 30.0 |
| Sodium chloride | 2.9 |
| Water | 67.0 |
| Sodium nitrite | 0.1 |
| | 100.0 |

## Example 14

### Shampoo

| | |
|---|---:|
| QUADROL-neutralized lauryl sulfate (40% active) | 60.0 |
| MONAMID 150 ADD | 5.0 |
| AMMONYX LO | 1.0 |
| SOLULAN 16 | 3.0 |
| Water | 30.9 |
| Bronopol | 0.1 |
| | 100.0 |

## Example 15

### Cream Lotion Shampoo

| | |
|---|---:|
| QUADROL-neutralized dodecyl benzene sulfonate (67% active) | 29.6 |
| Glyceryl monostearate | 3.0 |
| CARBOPOL 934 | 1.0 |
| QUADROL | 3.5 |
| Water | 62.8 |
| Sodium nitrite | 0.1 |
| | 100.0 |

0023978

VEEGUM is finely divided magnesium aluminum silicate.

Captan is $C_9H_8Cl_3NO_2S$, N-trichloromethylmer-captotetrahydraphthalimide.

QUADROL® is N,N,N',N'-tetrakis(2-hydroxypropyl)-ethylene diamine and is a trademark of BASF Wyandotte Corporation, Parsippany, N.J.

LAURETH -4 is an adduct resulting from the oxyethylation of lauryl alcohol with 4 moles of ethylene oxide.

LAURETH -23 is an adduct resulting from the oxyethylation of lauryl alcohol with 23 moles of ethylene oxide.

MYVACET 9-40 is distilled acetylated monoglycerides.

CARBOPOL 934 is a member of a group of water-soluble resins having excellent suspending, thickening and gel-forming properties ("Carbopol" is a trademark of the B. F. Goodrich chemical Co., Cleveland, Ohio).

AMERCHOL L 101® is one of a series of surface-active lanolin derivatives ("Amerchol" is a trademark of American Cholesteral Products, Inc., Edison, N.J.).

PEG 40 Stearate is the monostearic acid ester of a 40-unit polyoxyethylene glycol.

PPG 33 butyl ether is an adduct of butyl alcohol and 33 units of propylene oxide.

PPG 12 BUTETH 16 is a mixture of 12 moles of propylene oxide and 16 moles of ethylene oxide, condensed with butyl alcohol.

POLYSORBATE 60 is polyoxyethylene (20) sorbitan monostearate.

POLYPEPTIDE SF is a hydrolyzed animal protein product and is a trademark of Stepan Chemical Co.

PLURONIC 25R5 is a block polymer of the oxypropylene-oxyethylene-oxypropylene type disclosed in U.S. Patent No. 3,036,118, having an average molecular weight of 4500 and a proportion of the molecular weight attributable to the oxypropylene hydrophobe of approximately 50 percent.

MONAMID 150 ADD is a surface-active agent ("Monamid" is a trademark of Mona Industries, Inc., Paterson, New Jersey)

AMMONYX LO is dimethyl lauryl amine oxide ("Ammonyx" is a trademark of Onyx Chemical Co., Jersey City, New Jersey).

SOLULAN 16 is a 16-mole ethylene oxide adduct of lanolin alcohol ("Solulan" is a trademark of American Cholesterol Products, Inc., Edison, N.J.).

Bronopol is 2-bromo-2-nitropropane-1,3-diol.

While I have shown and described herein certain embodiments of my invention, I intend to cover as well any change or modification therein which may be made without departing from its spirit and scope.

Claims

1. A cosmetic or toiletry preparation of a kind hitherto made with the use of an alkanolamine ingredient selected from the group consisting of an alkanolamine selected from the group consisting of monoethanolamine, diethanolamine and triethanolamine and compounds resulting from the reaction of one of said alkanolamines with an acidic substance, said preparation containing in place of said alkanolamine, an appropriate amount of a totally hydroxyalkylated alkylene diamine containing 2 to 6 carbon atoms, said alkylene diamine having been oxyalkylated with an alkylene oxide containing at least 2 carbon atoms or a mixture of alkylene oxides containing 2 or more carbon atoms,

said oxyalkylated alkylene diamine being used in an amount which is, when a corresponding cosmetic or toiletry preparation would contain said alkanolamine, 80 to 200 percent by weight of the amount of alkanolamine present in such corresponding composition, and in the case in which said cosmetic or toiletry preparation contains a substance resulting from the reaction of alkanolamine with an acidic substance, there is used a proportion of oxyalkylated alkylene diamine which is approximately 80 to 200 percent of a stoichiometrically equivalent amount of triethanolamine.

2. A cosmetic or toiletry preparation as defined in claim 1, wherein said alkylene diamine is N,N,N',N'-tetrakis-(2-hydroxypropyl)ethylene diamine.

3.  A method of making a cosmetic or toiletry preparation of a kind which customarily contains in the free or neutralized form an alkanolamine selected from the group consisting of monoethanolamine, diethanolamine, and triethanolamine, characterized in that said method comprises the usual steps of compounding said preparation with the difference that for its non-carcinogenic effect an appropriate amount of a totally hydroxyalkylated alkylene diamine containing 2 to 6 carbon atoms, said alkylene diamine having been oxyalkylated with an alkylene oxide containing at least 2 carbon atoms or a mixture of alkylene oxides containing 2 or more carbon atoms, is used to replace said alkanolamine.

4.  A method as defined in claim 3, wherein said alkanolamine is triethanolamine and said alkylene diamine is N,N,N',N'-tetrakis(2-hydroxypropyl)ethylene diamine.

5.  A method as defined in claim 3, wherein said alkylene diamine is N,N,N',N'-tetrakis(2-hydroxypropyl)-ethylene diamine.

6.  A cosmetic or toiletry preparation of reduced nitrosamine content said preparation being of a kind hitherto made with the use of an alkanolamine ingredient selected from the group consisting of an alkanolamine selected from the group consisting of monoethanolamine, diethanolamine and triethanolamine and compounds resulting from the reaction of one of said alkanolamines with an acidic substance, said preparation containing in place of said alkanolamine, an appropriate amount of a totally hydroxyalkylated alkylene diamine containing 2 to 6 carbon atoms, said alkylene diamine having been oxyalkylated with an alkylene oxide containing at least 2 carbon atoms or a mixture of alkylene oxides containing 2 or more carbon atoms,

said oxyalkylated alkylene diamine being used in an amount which is, when a corresponding cosmetic or toiletry preparation would contain said alkanolamine, 80 to 200 percent by weight of the amount of alkanolamine present in such corresponding composition, and in the case in which said cosmetic or toiletry preparation contains a substance resulting from the reaction of alkanolamine with an acidic substance, there is used a proportion of oxyalkylated alkylene diamine which is approximately 80 to 200 percent of a stoichiometrically equivalent amount of triethanolamine.

7. A cosmetic or toiletry preparation as defined in claim 6, wherein said alkylene diamine is N,N,N',N'-tetrakis-(2-hydroxypropyl)ethylene diamine.

8. A cosmetic or toiletry preparation containing no detectable nitrosamines, said preparation being characterized by the use of ingredients containing no identified nitrosamine precursors and being of a kind hitherto made with the use of an alkanolamine ingredient selected from the group consisting of an alkanolamine selected from the group consisting of monoethanolamine, diethanolamine and triethanolamine and compounds resulting from the reaction of one of said alkanolamines with an acidic substance, said preparation containing in place of said alkanolamine, an appropriate amount of a totally hydroxyalkylated alkylene diamine containing 2 to 6 carbon atoms, said alkylene diamine having been oxyalkylated with an alkylene oxide containing at least 2 carbon atoms or a mixture of alkylene oxides containing 2 or more carbon atoms,

said oxyalkylated alkylene diamine being used in an amount which is, when a corresponding cosmetic or toiletry preparation would contain said alkanolamine, 80 to 200 percent by weight of the amount of alkanolamine present in such corresponding composition, and in the case in which said cosmetic or toiletry preparation contains a substance resulting from the reaction of alkanolamine with an acidic substance, there is used a proportion of oxyalkylated alkylene diamine which is approximately 80 to 200 percent of a stoichiometrically equivalent amount of triethanolamine.

9. A cosmetic or toiletry preparation as defined in claim 8, wherein said alkylene diamine is N,N,N',N'-tetrakis-(2-hydroxypropyl)ethylene diamine.